# FASCICULE DE BREVET EUROPEEN

(11) **EP 1 239 283 B1**
(45) Date de publication et mention de la délivrance du brevet: **01.12.2010**
(21) Numéro de dépôt: 02290387.6
(22) Date de dépôt: 18.02.2002
(51) Int. Cl.: G01N 33/50

(54) **Procédé pour l'identification et le comptage de cellules biologiques**
Verfahren zur Zell-Identifizierung und -Zählung
Method for the identification and counting of biological cells

(30) Priorité: 23.02.2001 FR 0102489
(43) Date de publication de la demande: 11.09.2002
(73) Titulaire: ABX, 34181 Montpellier Cedex 4 (FR)
(72) Inventeur: Lefevre, Didier, 34980 Saint Clement de Riviere (FR); Veriac, Sylvie, 34070 Montpellier (FR); Champseix, Henri, 34980 Saint Gely du Fesc (FR)
(74) Mandataire: Quantin, Bruno Marie Henri

(56) Documents cités:
- EP-A- 0 343 380
- FR-A- 2 759 166
- US-A- 5 232 857

## Description

L'invention se rapporte aux analyses biologiques et notamment aux analyses de sang.

Elle concerne plus particulièrement un réactif et un procédé pour l'identification et le comptage de cellules biologiques dans un échantillon, en particulier dans un échantillon de sang.

L'échantillon biologique peut être du sang humain ou animal, ou encore tout autre liquide biologique ou préparation biologique.

Dans le domaine des analyses biologiques, l'importance du diagnostic de la détermination et du comptage précis de différentes populations cellulaires est reconnue depuis longtemps. En effet, l'apparition de rapports d'équilibre anormaux entre populations cellulaires normales du sang peut être corrélée à l'apparition de certaines maladies, par exemple réactions immunitaires, inflammatoires, etc. De même, l'apparition de populations cellulaires anormales peut également être corrélée à l'apparition d'autres maladies, telles que des leucémies, etc.

Les méthodes traditionnelles d'analyse cytologique peuvent être variées et comprennent l'observation microscopique après coloration, et éventuellement sédimentation ou agrégation. La détermination automatique des cellules sanguines a commencé dans le début des années 1960 par la séparation des principales populations leucocytaires normales ; voir la référence bibliographique suivante : (1) Hallerman L., Thom R., Gerhartz H. : "Elektronische Differentialzählzung von Granulocyten und Lymphocyten nach intervaler Fluochromierung mit Acridinorange." Verh Deutsch Ges Inn Med 70 : 217, 1964.

La séparation des leucocytes a été réalisée en cytométrie de flux par l'utilisation de multiples principes impliquant les propriétés optiques et chimiques des cellules. Plusieurs automates d'hématologie ont été fabriqués, utilisant des techniques variées, comme le principe de Coulter pour la détermination des volumes, la mesure de lumière diffractée pour l'estimation des tailles, la mesure de lumière diffusée à 90° pour la détermination des structures internes des cellules, et les mesures de fluorescence ou d'absorption pour la détermination des affinités cellulaires à divers colorants ; voir les références bibliographiques 2 à 5 suivantes :
(2) Adams L.R., Kamensky L.A. : "Fluorometric Characterization of Six Classes of Human Leukocytes." Acta Cytol 18 : 389, 1974;
(3) Shapiro H.M. et al. "Combined Blood Cell Counting and Classification with Fluorochrome Stains and Flow Instrumentation" J. Histochem Cytochem 24 : 396-411, 1976 ;
(4) Terstappen L.W. et al. "Multidimensional Flow Cytometric Blood Cell Differentiation Without Erythrocyte Lysis." Blood Cells 17: 585-602, 1991 ;
(5) Terstappen L.W., Levin J. "Bone marrow cell differential counts obtained by multidimentional flow cytometry" Blood Cells 18 (2) : 311-30, 1992.

La caractérisation de cellules à des stades précoces du cycle cellulaire a depuis longtemps intéressé les scientifiques et la quantification du contenu de chaque cellule en ARN est reconnue de longue date comme étant un paramètre représentatif de ce cycle ; voir les références bibliographiques 2 à 5 ci-dessus et les références bibliographiques 6 et 7 suivantes :
(6) Traganos F., Darzynkiewicz Z., Sharpless T., Melamed M.R. "Simultaneous Staining of Ribonucleic and Deoxyribonucleic Acids in Unfixed Cells Using Acridine Orange in a Flow Cytofluorometric System" J. Histochem Cytochem 25 : 46, 1977 ;
(7) Pollack A. et al. "Flow Cytometric Analysis of RNA Content in Different Cell Populations Using Pyronin Y and Methyl Green" Cytometry, vol. 3, n° 1, pages 28-35, 1982.

Dans son brevet français n° 97 01090, du 31 janvier 1997, la demanderesse a décrit déjà une composition, et plus particulièrement un réactif de coloration, permettant ce type d'analyse.

Pour automatiser de telles techniques, il est nécessaire de résoudre préalablement de multiples problèmes comme, notamment, la réduction des temps et du coût de traitement des échantillons. Cette réduction peut être abordée de différentes manières, la plus évidente étant la réduction du nombre de canaux pour n'effectuer qu'une seule préparation cellulaire à la fois. Ce type de technique a préalablement été décrit par Léon W. Terstappen (référence 4 ci-dessus), mais nécessite un temps de traitement et d'analyse important, notamment pour le comptage précis des cellules nucléées dont la quantité est couramment mille fois plus faible que les cellules érythrocytaires.

Couramment, pour pallier cela, l'échantillon biologique est fréquemment séparé en au moins deux aliquotes, dont l'une est préparée à une certaine concentration permettant l'étude des cellules érythrocytaires et des plaquettes, et dont l'autre est préparée à une concentration plus forte pour l'analyse des cellules nucléées.

Ces techniques connues présentent différents inconvénients.

Préalablement à l'analyse, le traitement de cette aliquote comprend fréquemment la destruction spécifique des cellules érythrocytaires pour faciliter la mesure des cellules restantes. Une telle méthode permet d'obtenir plus rapidement les résultats des observations, mais est néanmoins freinée par les temps de réaction, de transfert et de coloration pour obtenir la préparation souhaitée.

Le temps d'incubation d'une suspension cellulaire dans une solution réactive est notamment lié au temps nécessaire aux principes actifs pour pénétrer à l'intérieur des cellules. Dans le brevet français 9701090 déjà cité, la demanderesse a décrit des moyens pour accélérer cette pénétration grâce à l'utilisation d'un additif, notamment d'un additif du type ionophore, pour aider à la pénétration cellulaire.

EP 0343380 utilise deux colorants pour le comptage de cellules biologiques nucléées.

Le temps de traitement est également fonction du nombre d'étapes successives que devra subir l'aliquote. La lyse et la coloration des cellules sont couramment réalisées en deux étapes successives, dans un ordre ou dans l'autre (voir le brevet US 6 004 816).

Ces deux étapes de dilution impliquent un coût non négligeable en matériel, associé à un temps de traitement minimal important.

US 5 232 857 utilise un colorant qui ne colore pas les acides nucléiques.

C'est en conséquence un but de proposer un réactif pour l'identification et le comptage de cellules biologiques qui surmonte les inconvénients précités.

C'est en particulier un but de procurer un tel réactif qui permet d'effectuer simultanément la lyse de certaines cellules, en particulier de cellules érythrocytaires, la fixation des cellules nucléées et la coloration du matériel intracellulaire.

C'est également un but de procurer un tel réactif qui permet de réaliser ces opérations dans un temps restreint pour réduire de façon importante les coûts et les temps d'analyse, et le nombre de réactifs.

On propose à cet effet un réactif pour l'identification et le comptage de cellules biologiques dans un échantillon, lequel comprend :
- un agent de lyse cellulaire choisi parmi au moins un détergent en une concentration efficace pour lyser spécifiquement un type donné de cellules de l'échantillon, et
- un colorant propre à marquer les acides nucléiques intracellulaires des cellules restantes non lysées.

On procure ainsi un réactif de lyse et de coloration simultanée d'un échantillon biologique, permettant d'obtenir en une seule étape une solution de cellules pouvant être analysée, par exemple par un système de cytométrie en flux. Cette analyse permet d'obtenir une classification et un comptage des cellules ainsi traitées.

Ainsi, le réactif combine une solution réactive du type décrit dans le brevet français 97 01090 avec un agent de lyse cellulaire qui permet de lyser spécifiquement un type donné de cellules de l'échantillon, en particulier les cellules érythrocytaires.

La solution réactive colorante en elle-même, décrite dans le brevet français 97 01090, permet d'accélérer la perméation membranaire pour la coloration des cellules biologiques de l'échantillon. Cette solution colorante peut être utilisée aussi bien avant qu'après la lyse des hématies selon les types cellulaires à étudier. Ainsi, le principe réactif de cette solution colorante a été conservé et transposé au sein d'une solution lytique permettant une destruction des hématies et une coloration des cellules restantes préalablement à la mesure.

L'agent de lyse cellulaire comprend avantageusement au moins un détergent ionique et/ou non ionique dans une concentration propre à lyser les érythrocytes.

Le détergent de l'invention est avantageusement choisi parmi :
- les amines primaires, les acétates et chlorhydrates d'amines, les sels d'ammonium quaternaire et le bromure de triméthylcéthyl ammonium ;
- les amides de diamines substituées, la diéthanolaminopropylamine ou le diéthylamino-propylamide, les amides de diéthylènetriamine cyclisés,
- les alkylaryl sulfonates, sulfonates de pétrole, glycérides sulfonés ;
- les cholamides, les sulfobétaïnes ;
- les alkyl glycosides, les saponines ;
- les polyoxyéthylène éthers et sorbitans, et les polyglycol éthers.

Dans un exemple de réalisation, ce détergent comprend un mélange de Triton X100 à une concentration de 0,05% (p/v) et de Tween 20 à une concentration de 0,0001% (v/v).

Dans toute la description, l'expression "p/v" signifie "poids/volume" et l'expression "v/v" signifie "volume/volume".

Le colorant utilisé est avantageusement du type fluorescent.

Avantageusement, on choisit un colorant qui est propre à s'associer spécifiquement à l'acide ribonucléique intracellulaire et à augmenter sa fluorescence, une fois associé à celui-ci.

Le colorant de l'invention peut être choisi notamment parmi la les colorants suivants :
- le thiazole orange ou 1-méthyl-4-[(3-méthyl-2-(3H)-benzothiazolylidène)méthyl]quinolinium p-tosylate,
- le thiazole blue,
- le quinolinium, 4-[(3-méthyl-2-(3H)-benzothiazolylidène)méthyl]-1-[3-(triméthylammonium)propyl], diodure,
- le 3,3'-diméthyloxacarbocyanine iodure ou 3-méthyl-2-[3-(3-méthyl-2(3H)-benzoxazolylidène)-1-propényl]benzoxazolium iodure,
- la thioflavine T,
- les colorants SYTO® et TOTO® (TM Molecular Probes),
- le bromure d'éthidium,
- l'iodure de propidium,
- l'acridine orange,
- la coriphosphine O,
- l'auramine O,
- les colorants HOECHST 33258 et HOECHST 33342,
- le 4',6-diamino-2-phénylindole, dihydrichlorure (DAPI),
- le 4',6-(diimidazolin-2-yl)-2-phénylindole, dihydrochlorure (DIPI),
- la 7 aminoactinomycine D,
- l'actinomycine D, et
- le LDS 751.

Dans une forme de réalisation préférée de l'invention, le réactif comprend en outre au moins un agent de pénétration membranaire propre à favoriser la pénétration du colorant dans les cellules à marquer.

L'agent favorisant la pénétration membranaire est avantageusement un composé ionophore de type protonophore et/ou antibiotique.

Cet agent est généralement présent à une concentration inférieure à 0,005% (p/v). Un exemple d'antibiotique utilisable est la valinomycine.

Il est avantageux que le réactif comprenne, en outre, au moins un agent de fixation membranaire présent à une concentration de 0,1% à 10% (p/v). Cet agent de fixation comprend, de préférence, au moins un alcool et/ou un aldéhyde. A ce titre, on préfère utiliser, par exemple, le paraformaldéhyde ou le glutaraldéhyde.

Il entre également dans le cadre de l'invention de prévoir d'autres additifs ou composants dans le réactif.

Ainsi, ce réactif peut comprendre, en outre, au moins un composé choisi parmi un agent complexant, un sel inorganique et un système tampon.

Sous un autre aspect, l'invention concerne un procédé pour l'identification et le comptage de cellules biologiques dans un échantillon, en particulier dans un échantillon de sang, lequel comprend les opérations suivantes :
- mélanger et incuber l'échantillon avec un réactif tel que défini ci-dessus pour réaliser, en une seule étape, la lyse de cellules d'un type donné, en particulier de cellules érythrocytaires, la coloration des acides nucléiques intracellulaires et la fixation des cellules nucléées ;
- mesurer la solution résultante en cytométrie de flux avec au moins deux paramètres de mesure choisis parmi le volume résistif, la diffraction lumineuse dans l'axe, la transmission lumineuse dans l'axe, la diffusion lumineuse orthogonale et la fluorescence ; et
- classifier et compter les cellules nucléées en populations, au moyens des paramètres mesurés.

Dans la mesure en cytométrie de flux, le paramètre de diffraction lumineuse dans l'axe est au moins un paramètre choisi parmi la diffraction aux petits angles et la diffraction aux grands angles.

Cette mesure peut être réalisée au moyen d'un cytomètre de flux possédant les paramètres conventionnels tels que la diffraction dans l'axe ou "FSC" (Forward Scatter), la diffusion orthogonale ou "SSC" (Side Scatter), la fluorescence soit orthogonale (FL1), soit dans l'axe, soit en épi-fluorescence, le tout polarisé ou dépolarisé, mais également des paramètres de mesure additionnels tels que la mesure de lumière transmise ou la volumétrie résistive, telles que décrites dans le brevet français 89 14120 du 27 octobre 1989.

La résistivité peut être mesurée au moyen d'un courant continu (DC) afin d'exprimer le volume des éléments et/ou au moyen d'un courant pulsé ou alternatif (RF) afin d'exprimer les différences densimétriques internes s'approchant de la détermination de la structure.

De ces paramètres, peuvent être extraits des ensembles d'informations multiparamétriques pour chacune des cellules analysées, permettant leur classification. La classification sera d'autant plus précise que les paramètres définissant les cellules seront pertinents et nombreux. Ce type d'étude multiparamétrique a déjà été décrit précédemment (voir les références bibliographiques 4 et 5 ci-dessus).

Dans le cadre de l'invention, les cellules nucléées classifiées sont indifféremment des cellules matures ou immatures, normales ou anormales.

La classification des cellules nucléées est effectuée par des procédés connus. Elle peut être réalisée par un logiciel d'analyse multidimensionnel avec ou sans le recours à une technique neuronale logicielle ou non.

Dans le cadre de l'invention, l'échantillon biologique peut être un échantillon de sang humain ou animal, ou encore un échantillon de liquide biologique ou une suspension de cellules, d'origine humaine ou animale.

Cet échantillon est mélangé avec la solution réactive dans des conditions de température définies. La cinétique de réaction fait que les cellules érythrocytaires sont d'abord détruites, la pénétration du colorant est aidée parallèlement à la fixation des cellules qui se fait plus lentement.

L'invention sera décrite maintenant en référence à l'exemple suivant :

### Exemple

Dans le cadre de cet exemple, on utilise un réactif ayant la composition suivante :

| | | | |
|---|---|---|---|
| Agent complexant | EDTA | 0,02 % | (p/v) |
| Sel inorganique | NaCl | 0,85 % | (p/v) |
| Système tampon | Phosphates | 0,5 % | (p/v) |
| Détergents | Triton X100 | 0,05 % | (p/v) |
| | Tween 20 | 0,0001 % | (v/v) |
| Ionophore | Valinomycine | 0,003 % | (p/v) |
| Colorant | Thiazole orange | 0,005 % | (p/v) |
| Aldéhyde | Paraformaldéhyde | 1 % | (p/v) |

Un échantillon de sang total est mélangé à la solution réactive ci-dessus. Après une incubation de quelques secondes (typiquement de l'ordre de 15 à 30 secondes), la solution est analysée au moyen d'un ensemble de cytométrie de flux comprenant au moins les paramètres suivants : diffraction dans l'axe (FSC) donnant une interprétation de la taille, diffusion orthogonale (SSC) exprimant la structure des éléments observés et fluorescence orthogonale (FL1) permettant de mesurer l'expression de l'acide ribonucléique intracellulaire.

Les résultats ainsi obtenus sont observés en mode multidimensionnel, afin de déterminer les inter-relations des différentes populations entre chaque paramètre.

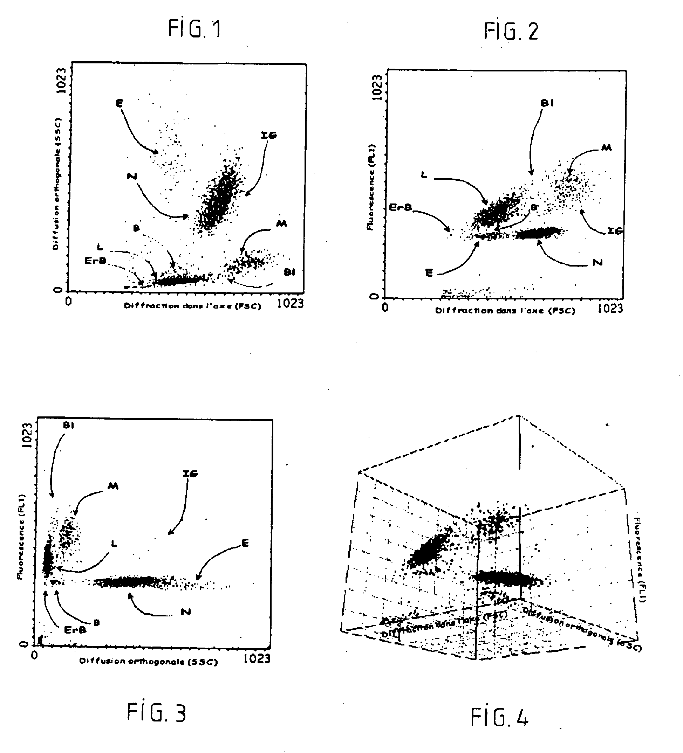

On se réfère maintenant aux Figures 1 à 4 qui représentent les résultats obtenus avec un échantillon de sang humain normal.

La Figure 1 représente la matrice obtenue au moyen des deux paramètres de diffraction dans l'axe (FSC) et de diffusion orthogonale (SSC). Quatre populations se détachent nettement : L figurant les lymphocytes, M figurant les monocytes, N figurant les polynucléaires neutrophiles et E figurant les polynucléaires éosinophiles. Les populations IG pour immature granuleux, BL pour blastes, B pour polynucléaires basophiles et ErB pour érythroblastes sont indiquées mais non dissociables en seulement deux dimensions.

La Figure 2 représente la matrice formée par les paramètres de diffraction dans l'axe (FSC) et de fluorescence (FL1). Les quatre mêmes populations que représenté à la Figure 1 se retrouvent, mais organisées différemment. Les cellules mononucléées L et M forment le groupe supérieur de fluorescence moyenne et les cellules polymorphonucléées N, E et B forment le groupe inférieur de fluorescence faible. La population ErB des érythroblastes est nettement séparée à la pointe des deux groupes ainsi formés. Les emplacements normaux des populations BL et IG sont indiqués.

La Figure 3 représente la matrice formée par les paramètres de diffusion orthogonale (SSC) et de fluorescence (FL1). Les mêmes populations se retrouvent organisées d'une manière différente mais permettant d'isoler les populations IG et BL (en très petites quantités dans un échantillon normal).

La Figure 4 représente une vision tridimensionnelle des populations obtenues.

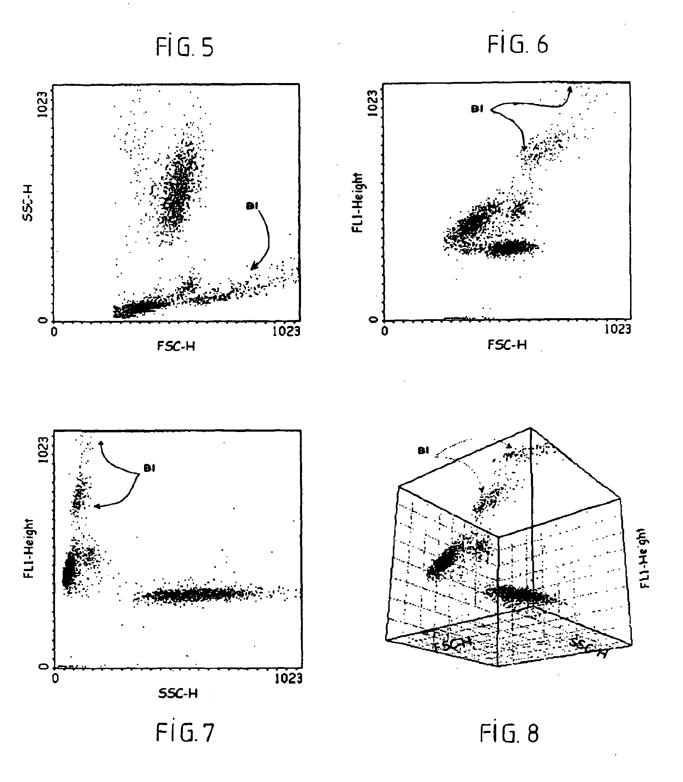

Les Figures 5 à 8 représentent les mêmes types de résultats que les Figures 1 à 4 respectivement, mais obtenus avec un échantillon présentant des cellules blastiques (B1) et traité conformément à l'invention.

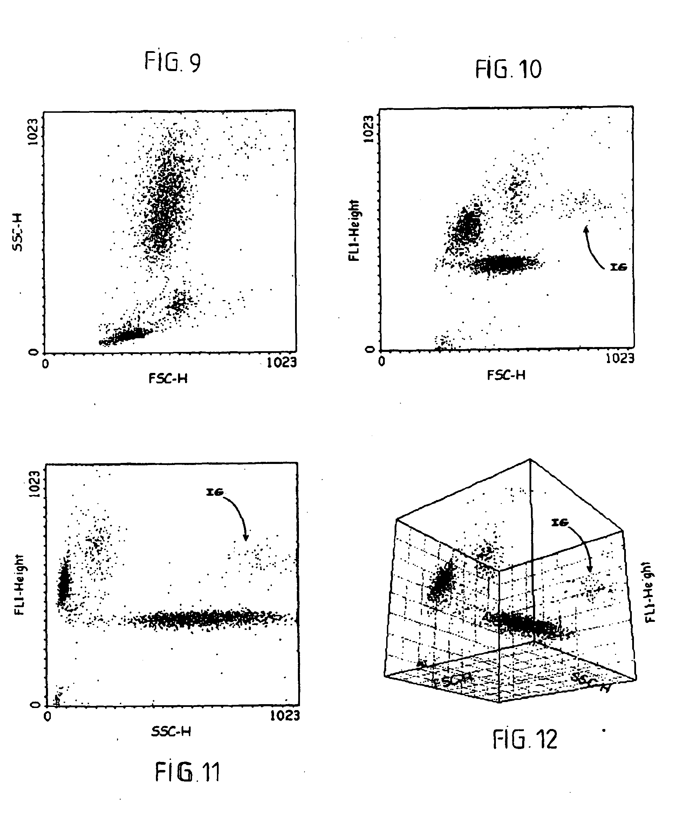

Les Figures 9 à 12 représentent les mêmes types de résultats que les Figures 1 à 4 respectivement, mais obtenus avec un échantillon présentant des cellules granulocytaires immatures (IG) et traité conformément à l'invention.

Le réactif et le procédé de l'invention permettent ainsi, en une seule étape, de réaliser une lyse spécifique et une coloration simultanée de cellules biologiques dans un échantillon, en particulier dans un échantillon de sang humain ou animal.

On peut ainsi obtenir rapidement une identification et un comptage de cellules à partir d'un automate d'analyse basé sur la cytométrie de flux.

## Revendications

1. Procédé pour l'identification et le comptage de cellules biologiques nucléées dans un échantillon, en particulier dans un échantillon de sang, **caractérisé en ce qu'**il comprend les opérations suivantes ;
- mélanger et incuber l'échantillon avec un réactif comprenant 1) un agent de lyse cellulaire choisi parmi au moins un détergent ionique et/ou non ionique dans une concentration propre à lyser les érythrocytes choisi parmi les amines primaires, les acétates et chlorhydrates d'amines, les sels d'ammonium quaternaire et le bromure de triméthylcéthyl ammonium ; les amides de diamines substituées, la diéthanolaminopropylamine ou le diéthylamino-propylamide, les amides de diéthylénetriamine cyclisés, les glycérides sulfonés ; les saponines, et 2) un colorant propre à marquer les acides nucléiques intracellulaires des cellules restantes non lysées, pour réaliser, en une seule étape, la lyse de cellules d'un type donné, en particulier de cellules érythrocytaires, la coloration des acides nucléiques intracellulaires et la fixation des cellules nucléées ;
- mesurer la solution résultante en cytométrie de flux avec au moins deux paramètres de mesure choisis parmi le volume résistif, la diffraction lumineuse dans l'axe, la transmission lumineuse dans l'axe, la diffusion lumineuse orthogonale et la fluorescence ; et
- classifier et compter les cellules nucléées en populations, au moyen des paramètres mesurés,

2. Procédé selon la revendication 1, **caractérise en ce que** le colorant est du type fluorescent.

3. Procédé selon l'une des revendications 1 ou 2, **caractérisé en ce que** le colorant est propre à s'associer spécifiquement à l'acide ribonucléique intracellulaire et à augmenter sa fluorescence, une fois associé à celui-ci.

4. Procédé selon l'une des revendications 1 à 3, **caractérisé en ce que** le colorant est choisi parmi:
le thiazole orange ou 1-méthyl-4-[(3-méthyl-2-(3H)-benzothiazolylidène)méthyl]quinolinium p-tosylate,
le thiazole blue,
le quinolinium, 4-[(3-méthyl-2-(3H)-benzothiazolylidène)méthyl]-1-[3-(triméthylammonium)propyl], diodure,
le 3,3'-diméthyloxacarbocyanine ioduré ou 3-méthyl-2-[3-(3-méthyl-2(3H)-benzoxazolylidène)-1-propényl]benzoxazolium iodure,
la thioflavine T,
les colorants SYTO TM et TOTO TM (TM Molecular Probes),
le bromure d'éthidium,
l'iodure de propidium,
l'acridine orange,
la coriphosphine O,
l'auramine O,
les colorants HOECHST 33258 et HOECHST 33342,
le 4',6-diamino-2-phénylindole, dihydrichlorure (DAPI),
le 4',6-(diimidazolin-2-yl)-2-phénylindole, dihydrochlorure (DIPI),
la 7 aminoactinomycine D,
l'actinomycine D, et
le LDS 751.

5. Procédé selon l'une des revendications 1 à 4, **caractérisé en ce que** le réactif comprend en outre au moins un agent de pénétration membranaire propre à favoriser la pénétration du colorant dans les cellules à marquer.

6. Procédé selon la revendication 5, **caractérisé en ce que** l'agent favorisant la pénétration membranaire est un composé ionophore de type protonophore et/ou antibiotique.

7. Procédé selon l'une des revendications 1 à 6, **caractérisé en ce que** le réactif comprend en outre au moins un agent de fixation membranaire présent à une concentration de 0,1% à 10% (p/v).

8. Procédé selon la revendication 7, **caractérisé en ce que** l'agent de fixation membranaire comprend au moins un alcool et/ou un aldéhyde choisi parmi le paraformaldéhyde et le glutaraldéhyde.

9. Procédé selon l'une des revendications 1 à 8, **caractérisé en ce que** le réactif comprend en outre au moins un composé choisi parmi un agent complexant, un sel inorganique et un système tampon.

10. Procédé selon l'une des revendications 1 à 9, **caractérisé en ce que** la mesure de résistivité est effectuée au moyen d'au moins un courant choisi parmi un courant continu (DC) et un courant pulsé ou alternatif (RF).

11. Procédé selon l'une des revendications 1 à 10, **caractérisé en ce que** le paramètre de diffraction lumineuse dans l'axe est au moins un paramètre choisi parmi la diffraction aux petits anglets et la diffraction aux grands angles.

12. Procédé selon l'une des revendications 1 à 11, **caractérisé en ce que** les cellules nucléées classifiées sont indifféremment des cellules matures ou immatures, normales ou anormales.

13. Procédé selon l'une des revendications 1 à 12, **caractérisé en ce que** la classification des cellules nucléées est réalisée par un logiciel d'analyse multidimensionnel avec ou sans le recours à une technique neuronale logicielle ou non.

14. Procédé selon l'une des revendications 1 à 13, **caractérisé en ce que** l'échantillon est un échantillon de sang humain ou animal.

15. Procédé selon l'une des revendications 1 à 14, **caractérisé en ce que** l'échantillon est un échantillon de liquide biologique ou une suspension de cellules, d'origine humaine ou animale.

## Claims

1. Process for the identification and counting of nucleated biological cells in a sample, in particular in a blood sample, **characterized in that** it comprises the following operations:
- mixing and incubating the sample with a reagent comprising 1) a cell lysing agent chosen from at least one ionic and/or non-ionic detergent in a concentration suitable for lysing erythrocytes, chosen from the primary amines, amine acetates and hydrochlorides, quaternary ammonium salts and trimethylethyl ammonium bromide; substituted diamine amides, diethanolaminopropylamine or diethylaminopropylamide, cyclized diethylene triamine amides, sulphonated glycerides; saponins, and 2) a dye suitable for labelling the intracellular nucleic acids of the cells remaining unlysed, in order to carry out, in a single stage, the lysis of cells of a given type, in particular erythrocyte cells, the staining of the intracellular nucleic acids and the binding of the nucleated cells;
- measuring the resultant solution in flow cytometry with at least two measurement parameters chosen from resistive volume, on-axis light diffraction, on-axis light transmission, orthogonal light diffusion and fluorescence; and
- classifying and counting the nucleated cells in populations, by means of the measured parameters.

2. Process according to claim 1, **characterized in that** the dye is of the fluorescent type.

3. Process according to one of claims 1 or 2, **characterized in that** the dye is suitable for binding specifically to the intracellular ribonucleic acid and increasing its fluorescence, once bound to the latter.

4. Process according to one of claims 1 to 3, **characterized in that** the dye is chosen from:
thiazole orange or 1-methyl-4-[(3-methyl-2(3H)-benzothiazolylidene)methyl]quinolinium p-tosylate,
thiazole blue,
4-[(3-methyl-2-(3H)-benzothiazolyl-idene)methyl]-1-[3-(trimethylammonium)propyl] quinolinium diiodide,
3,3'-dimethyloxacarbocyanine iodide or 3-methyl-2-[-3-(3-methyl-2(3H)-benzoxazolylidene)-1-propenyl]benzoxazolium iodide,
thioflavin T,
the dyes SYTO TM and TOTO TM (TM Molecular Probes),
ethidium bromide,
propidium iodide,
acridine orange,
coriphosphine-O,
auramine O,
the dyes HOECHST 33258 and HOECHST 33342,
4'-6-diamino-2-phenylindole dihydrochloride (DAPI),
4'-6-(diamidazolin-2-yl)-2-phenylindole dihydrochloride (DAPI),
7-aminoactinomycin D,
actinomycin D, and
LDS 751.

5. Process according to one of claims 1 to 4, **characterized in that** the reagent also comprises at least one membrane penetration agent capable of promoting the penetration of the dye into the cells to be labelled.

6. Process according to claim 5 **characterized in that** the agent promoting membrane penetration is an ionophore compound of the protonophore and/or antibiotic type.

7. Process according to one of claims 1 to 6, **characterized in that** it also comprises at least one membrane fixing agent present in a concentration of 0.1 % to 10% (w/v).

8. Process according to claim 7, **characterized in that** the membrane fixing agent comprises at least one alcohol and/or an aldehyde selected from paraformaldehyde and glutaraldehyde.

9. Process according to one of claims 1 to 8, **characterized in that** it also comprises at least one compound selected from a complexing agent, an inorganic salt and a buffer system.

10. Process according to one of claims 1 to 9, **characterized in that** the resistivity measurement is carried out by means of at least one current chosen from a continuous current (DC) and a pulsed or alternating current (RF).

11. Process according to one of claims 1 to 10, **characterized in that** the on-axis light diffraction parameter is at least one parameter chosen from small angle diffraction and large angle diffraction.

12. Process according to one of claims 1 to 11, **characterized in that** the nucleated cells classified are indiscriminately mature or immature, normal or abnormal cells.

13. Process according to one of claims 1 to 12, **characterized in that** the classification of the nucleated cells is carried out by multidimensional analysis software with or without use of a software neural network technique.

14. Process according to one of claims 1 to 13, **characterized in that** the sample is a human or animal blood sample

15. Process according to one of claims 1 to 14, **characterized in that** the sample is a sample of biological fluid or a suspension of cells of human or animal origin.

## Patentansprüche

1. Verfahren zum Identifizieren und Zählen von nukleierten biologischen Zellen in einer Probe, insbesondere in einer Blutprobe, **dadurch gekennzeichnet, dass** es die folgenden Schritte beinhaltet:
- Mischen und Inkubieren einer Probe mit einem Reaktionsmittel, das Folgendes enthält: 1) ein Zelllysemittel, ausgewählt aus wenigstens einem ionischen und/oder nichtionischen Detergenz in einer Konzentration, die zum Lysieren der Erythrozyten geeignet ist, ausgewählt aus primären Aminen, Acetaten und Aminchlorhydraten, quaternären Ammoniumsalzen und Trimethylethyl-Ammoniumbromid; substituierten Diaminamiden, Diethanolaminopropylamin oder Diethylaminopropylamid, zyklisierten Diethylentriaminamiden, sulfonierten Glyceriden; Saponinen, und 2) einen Farbstoff, der zum Markieren der intrazellulären Nukleinsäuren der restlichen nichtlysierten Zellen geeignet ist, um in einem einzigen Schritt die Lyse von Zellen eines gegebenen Typs, insbesondere von erythrozytären Zellen, die Färbung von intrazellulären Nukleinsäuren und die Fixierung der nukleierten Zellen durchzuführen;
- Messen der resultierenden Lösung durch Flusszytometrie mit wenigstens zwei Messparametern, ausgewählt aus Widerstandsvolumen, Lichtbeugung auf der Achse, Lichttransmission auf der Achse, orthogonale Lichtdiffusion und Fluoreszenz; und
- Klassifizieren und Zählen der nukleierten Zellen in Populationen anhand der gemessenen Parameter.

2. Verfahren nach Anspruch 1, **dadurch gekennzeichnet, dass** der Farbstoff vom fluoreszierenden Typ ist.

3. Verfahren nach Anspruch 1 oder 2, **dadurch gekennzeichnet, dass** der Farbstoff geeignet ist, sich speziell an die intrazelluläre Ribonukleinsäure zu binden und nach der Bindung an diese deren Fluoreszenz zu erhöhen.

4. Verfahren nach einem der Ansprüche 1 bis 3, **dadurch gekennzeichnet, dass** der Farbstoff ausgewählt ist aus:
Thiazol Orange oder 1-Methyl-4-[(3-methyl-2(3H)-benzothiazolyliden)methyl]chinolinium-p-Tosylat,
Thiazol Blue,
4-[(3-Methyl-2(3H)-benzothiazolyliden)methyl]-1-[3-(trimethylammonium)propyl]-Chinolinium diiodid,
3,3'-Dimethyloxacarbocyaniniodid oder 3-Methyl-2-[3-(3-methyl-2(3H)-benzoxazolyliden)-1-propenyl]benzoxazoliumiodid,
Thioflavin T,
die Farbstoffe SYTO TM und TOTO TM (TM Molekulare Sonden), Ethidiumbromid,
Propidiumiodid,
Acridin Orange
Coriphosphin O,
Auramin O,
die Farbstoffe HOECHST 33258 und HOECHST 33342,
4',6-Diamino-2-phenylindol-dihydrochlorid (DAPI),
4', 6-(Diimidazolin-2-yl)-2-phenylindol-dihydrochlorid (DIPI),
7-Aminoactinomycin D,
Actinomycin D, und
LDS 751.

5. Verfahren nach einem der Ansprüche 1 bis 4, **dadurch gekennzeichnet, dass** das Reaktionsmittel darüber hinaus wenigstens ein Membranpenetrationsmittel enthält, das die Penetration des Farbstoffs in die zu markierenden Zellen begünstigt.

6. Verfahren nach Anspruch 5, **dadurch gekennzeichnet, dass** das die Membranpenetration begünstigende Mittel eine ionophore Verbindung des protonophoren und/oder antibiotischen Typs ist.

7. Verfahren nach einem der Ansprüche 1 bis 6, **dadurch gekennzeichnet, dass** das Reaktionsmittel darüber hinaus wenigstens ein Membranfixiermittel beinhaltet, das in einer Konzentration von 0,1 bis 10% (p/v) vorliegt.

8. Verfahren nach Anspruch 7, **dadurch gekennzeichnet, dass** das Membranfixiermittel wenigstens einen Alkohol und/oder ein Aldehyd umfasst, das ausgewählt ist aus Paraformaldehyd und Glutaraldehyd.

9. Verfahren nach einem der Ansprüche 1 bis 8, **dadurch gekennzeichnet, dass** das Reaktionsmittel darüber hinaus wenigstens eine Verbindung umfasst, die ausgewählt ist aus einem Komplexbildner, einem anorganischen Salz und einem Puffersystem.

10. Verfahren nach einem der Ansprüche 1 bis 9, **dadurch gekennzeichnet, dass** die Widerstandsmessung mithilfe von wenigstens einem Farbstoff erfolgt, ausgewählt aus Gleichstrom (DC) und Impuls- oder Wechselstrom (RF).

11. Verfahren nach einem der Ansprüche 1 bis 10, **dadurch gekennzeichnet, dass** der Parameter der Lichtbeugung auf der Achse wenigstens ein Parameter ist, der ausgewählt ist aus Brechung in kleinen Winkeln und Brechung in großen Winkeln.

12. Verfahren nach einem der Ansprüche 1 bis 11, **dadurch gekennzeichnet, dass** die klassifizierten nukleierten Zellen von reifen oder unreifen, normalen oder anormalen Zellen nicht zu unterscheiden sind.

13. Verfahren nach einem der Ansprüche 1 bis 12, **dadurch gekennzeichnet, dass** die Klassifikation der nukleierten Zellen durch eine mehrdimensionale Analysesoftware mit oder ohne Rückgriff auf eine neuronale Softwaretechnik erfolgt.

14. Verfahren nach einem der Ansprüche 1 bis 13, **dadurch gekennzeichnet, dass** die Probe eine menschliche oder tierische Blutprobe ist.

15. Verfahren nach einem der Ansprüche 1 bis 14, **dadurch gekennzeichnet, dass** die Probe eine Probe einer biologischen Flüssigkeit oder einer Zellsuspension menschlichen oder tierischen Ursprungs ist.
